# EUROPÄISCHE PATENTANMELDUNG

(11) **EP 4 484 151 A1**
(43) Veröffentlichungstag der Anmeldung: **01.01.2025**
(21) Anmeldenummer: 23193299.7
(22) Anmeldetag: 24.08.2023
(51) Int. Cl.: B32B 3/04, A61G 1/01, A61G 1/044, A61G 1/048, B32B 3/08, B32B 5/18, B32B 15/04, B32B 27/06

(54) **MULTIFUNKTIONSPAD**

(30) Priorität: 28.06.2023 DE 202023103587 U
(71) Anmelder: Feicht, Holger, 93183 Kallmünz (DE)
(72) Erfinder: Feicht, Holger, 93183 Kallmünz (DE)
(74) Vertreter: Bittner, Bernhard

(57) **Zusammenfassung**

Multifunktionspad (1), insbesondere für Feuerwehren, Rettungsdienste und dergleichen mit einem Innenkörper (2) der sich in einer Längsrichtung (L), einer zu der Längsrichtung senkrecht stehenden Breitenrichtung (B) und einer zu der Längsrichtung und der Breitenrichtung senkrecht stehenden Dickenrichtung erstreckt, wobei die Erstreckung in der Längsrichtung und die Erstreckung in der Breitenrichtung wesentlich größer ist als die Erstreckung in der Dickenrichtung, wobei das Multifunktionspad eine erste Außenoberfläche (1a) und eine zweite Außenoberfläche (1b) aufweist und die zweite Außenoberfläche (1b) der erste Außenoberfläche (1a) gegenüber liegt und wobei dieser Innenkörper wenigstens teilweise von einem flexiblen Bezug (4) umhüllt ist, dadurch gekennzeichnet, dass der Innenkörper (2) mehrschichtig aufgebaut ist und einen Schaumstoffkern (22) aufweist, der sich ebenfalls in der Längsrichtung (L), der Breitenrichtung (B) und der Dickenrichtung (D) erstreckt und der eine erste Außenoberfläche (22a) und eine zweite Außenoberfläche (22b) aufweist, wobei die erste Außenoberfläche (22a) der zweiten Außenoberfläche (22b) gegenüberliegt und der Innenkörper weiterhin einen ersten Schaumstoffkörper (24) sowie eine zwischen diesem Schaumstoffkern (22) und dem ersten Schaumstoffkörper (24) angeordnete erste Zwischenschicht (26) aus einem Kunststoff- oder Metallmaterial aufweist.

## Beschreibung

Die vorliegende Erfindung bezieht sich auf ein Multifunktionspad, insbesondere für Krisenfälle, das Rettungswesen, der Polizei, Justiz, Zoll, THW, Militär, Wasserrettung, Feuerwehrwesen und die erste Hilfe. Ebenso im Personennah- und Fernverkehr zu Wasser, Land und Luft. In diesen genannten Gebieten besteht oftmals ein hoher Bedarf an unterschiedlichen Hilfsmitteln, etwa zum Lagern verletzter Personen oder zum Bergen von Personen aber auch zur Gefahrenabwehr beispielsweise bei Demonstrationen, Unfällen oder Unglücken.

Im Stand der Technik ist es üblich, zu diesen unterschiedlichen Zwecken unterschiedliche Gerätschaften einzusetzen, die je nach Bedarfsfall auch in Einsatzfahrzeugen mitzuführen sind.

Der vorliegenden Erfindung liegt die Aufgabe zugrunde, eine Einrichtung zu schaffen, die für möglichst viele unterschiedliche Einsatzgebiete verwendbar ist, so dass auf das Mitführen einer hohen Anzahl an unterschiedlichen Gegenständen verzichtet werden kann. Bevorzugt soll diese Einrichtung weiterhin bequem von einem Nutzer gehalten und getragen werden können.

Diese Aufgaben werden erfindungsgemäß durch den Gegenstand des unabhängigen Anspruchs erreicht. Vorteilhaften Ausführungsformen und Weiterbildungen sind Gegenstand der Unteransprüche.

Ein erfindungsgemäßes Multifunktionspad, insbesondere für Feuerwehren, Rettungsdienste und dergleichen weist einen Innenkörper auf der sich in einer Längsrichtung, einer zu der Längsrichtung senkrecht stehenden Breitenrichtung und einer zu der Längsrichtung und der Breitenrichtung senkrecht stehenden Dickenrichtung erstreckt, wobei die Erstreckung in der Längsrichtung und die Erstreckung in der Breitenrichtung wesentlich größer ist als die Erstreckung in der Dickenrichtung (bevorzugt wenigstens doppelt, bevorzugt wenigstens dreimal so groß und bevorzugt wenigstens viermal so groß), wobei das Multifunktionspad eine erste Außenoberfläche und eine zweite Außenoberfläche aufweist und die zweite Außenoberfläche der ersten Außenoberfläche gegenüber liegt und wobei dieser Innenkörper wenigstens teilweise von einem flexiblen Bezug umhüllt ist.

Bevorzugt ist der Bezug bezüglich einer Achse, die sich in der Längsrichtung durch den Innenkörper erstreckt vollständig um den Innenkörper herum angeordnet. Bevorzugt ist der Innenkörper durch den Bezug vollständig eingeschlossen.

Erfindungsgemäß ist der Innenkörper mehrschichtig aufgebaut und weist einen Schaumstoffkern auf, der sich ebenfalls in der Längsrichtung, der Breitenrichtung und der Dickenrichtung erstreckt und der eine erste Außenoberfläche und eine zweite Außenoberfläche aufweist, wobei die erste Außenoberfläche der zweiten Außenoberfläche gegenüberliegt (und bevorzugt die erste Außenoberfläche und die zweite Außenoberfläche wenigstens abschnittsweise zueinander parallel sind) und der Innenkörper weiterhin einen ersten Schaumstoffkörper sowie eine zwischen diesem Schaumstoffkern und dem ersten Schaumstoffkörper angeordnete erste Zwischenschicht aus einem Kunststoffmaterial und/oder einem textilen Material und/oder Metallmaterial aufweist.

Die hier erwähnten Außenoberflächen des Innenkörpers sind auf diesen Innenkörper bezogen und sind bevorzugt keine Außenoberflächen des Multifunktionspads.

Bevorzugt verlaufen jedoch die Außenoberflächen des Innenkörpers parallel zu Außenoberflächen des Multifunktionspads. Besonders bevorzugt verläuft die Zwischenschicht parallel zu der Außenoberfläche des Innenkörpers.

Bei einer weiteren bevorzugten Ausführungsform besteht Zwischenschicht aus Metall. Besonders bevorzugt handelt es sich bei dieser Zwischenschicht um eine Aluplatte bzw. ein

Alublech. Die Zwischenschicht könnte jedoch auch aus einem Kunststoff hergestellt sein. Bevorzugt weist die Zwischenschicht eine geringere Flexibilität auf als der Schaumstoffkern. Bevorzugt ist eine Dicke des Schaumstoffkerns wenigstens 4 mal so groß wie eine Dicke der Zwischenschicht, bevorzugt wenigstens 6 mal so groß und bevorzugt wenigstens 10mal so groß-
Bei einer bevorzugten Ausführungsform ist eine Dicke dieser (Zwischen)Schicht wenigstens 0,1 mm, bevorzugt wenigstens 0,2 mm, bevorzugt 0,3 mm und bevorzugt wenigstens 0,4 mm und bevorzugt wenigstens 0,5 mm.

Bei einer bevorzugten Ausführungsform ist eine Dicke dieser Schicht höchstens 4,0 mm, bevorzugt höchstens 3,5 mm, bevorzugt höchstens 3,0mm, bevorzugt höchstens 2,5 mm, bevorzugt höchstens 2,0 mm, bevorzugt höchstens 1,5 mm und besonders bevorzugt höchstens 1,0 mm.

Bei einer bevorzugten Ausführungsform ist das Multifunktionspad tragbar und insbesondere von einer einzigen Person tragbar.

Das hier beschriebene Multifunktionspad kann für die unterschiedlichsten Einsatzgebiete zur Anwendung kommen.

So kommt eine Anwendung als Unterlage für Schocklageposition in Betracht (Auflage der Unterschenkel beispielsweis stirnseitig, wobei das Multifunktionspad fixierbar ist und insbesondere zwischen Schenkel und Boden fixierbar ist, wobei die Fixierung beispielsweise durch die Schwerkraft erfolgt).

Weiterhin ist eine Anwendung als Unterlage zur Überstreckung des Kopfes bei Erster Hilfe möglich (beispielsweise eine Fixierung zur Befreiung des Atemkanals vor Fremdkörpern)

Weiterhin kann das Multifunktionspad, im Folgenden auch nur kurz als Pad bezeichnet als Unterlage zur Durchführung der Beatmung verwendet werden (beispielsweise als Kopfauflage stirnseitig zur Kopfstütze oder das Pad seitig).

Eine weitere Anwendung kann ein Schutz der Knie bei Rettern und Einsatzkräften sein (beispielsweise Vermeidung von Langzeitschäden und Berufskrankheiten, Schutz älterer Personen und/oder Arbeitsschutz).

Eine weitere Anwendung dient der effektiveren Durchführung der Herzdruckmassage aufgrund der höheren Position ca. 3 - 4 cm und des verbesserten Druckwinkels von ca. 30 Grad.

Bei einer bevorzugten Ausführungsform kann das Pad eine integrierte wechselbare Anleitung etwa für Beatmung und Herzdruckmassage aufweisen (insbesondere für Retter welche keine Ausbildung als Retter haben).

Bei einer weitere bevorzugten Ausführungsform weist das Pad wenigstens eine integrierte Tasche für Notfallausstattung (beispielsweise für eine Rettungsdecke, ein Beatmungstuch und/oder medizinische Handschuhe) auf.

Bei einer weiteren bevorzugten Ausführungsform weist das Pad Patches mit Reflektionsstreifen zur besseren Sichtbarkeit bei Dunkelheit, Nebel, Regen, Schnee auf welche beispielsweise mittels Klett am Pad befestigt werden und dadurch vorteilhaft austauschbar ersetzbar sind. Diese Pads und/oder Patches können mittels Druckverfahren beschriftet werden

Bevorzugt ist auch eine Anwendung als sichere Ablage zur Kleinkindrettung möglich, diese können dabei umschlossen von einer Rettungsdecke sein. Vorzugsweise können auch ein oder mehrere Gurte vorgesehen sein.

Ein weiteres Anwendungsbeispiel ist die Abwehr von Angriffen mit stumpfen Gegenständen (Schläge, Tritte, Stöcke etc.). Daneben ist aber auch eine Anwendung zur Abwehr von Angriffen mit Stichwaffen oder spitzen Gegenständen (z. B. Messer, Spritzen) integriert in Anlehnung an KDIW 2004 min SK1und ein optionaler zusätzlicher wahlweiser ballistischer Schutz nach VPAM3+ 9mm Pistole und Maschinenpistole möglich-
Daneben kommt auch eine Anwendung in der Verunfalltenbetreuung beispielsweise als Schonfunktion für Knie oder Gesäß des Retters vor Nässe, Kälte, Hitze, Spitzen Kanten oder Steinen in Betracht.

Auch ist eine Anwendung als Unterlage für den Kopf bei eingeklemmten oder verschütteten Personen denkbar.

Auch kommt eine Anwendung zum Einsatz bei der Wasserrettung See und Salzwasser in Betracht. Zu diesem Zweck kann das Pad eine integrierte Stautasche und/oder ein integriertes Seil aufweisen, etwa ein schwimmfähiges Rettungsseil in einer bestimmen Länge, beispielsweise 20m.

Es ist weiterhin auch eine Anwendung zum Einsatz bei der Eisrettung als Gleitschlitten oder als Rettungshilfe denkbar (dabei kann der Oberkörper aus dem Wasser ragen und es ist eine erleichterte Atmung möglich). Wahlweise kann das Pad als Spinboard zur Rettung eingeklemmter Personen angewendet werden. Die Person sitzt dann auf einem Sitz, dazwischen das Pad und kann mittels z. B. Kran aus dem Fahrzeugsitz gerettet werden.

Bei einer weiteren bevorzugten Ausführungsform weist das Pad integrierbare Aufbewahrungstaschen für diverse weitere Rettungsutensilien (Leuchten,

Signalpfeiffe etc.) auf. Ebenso können weitere Aufbewahrungstaschen für Rettungsmittel per Steckadaptersystem konfektioniert und am Pad befestigt werden.

Bei einer weiteren Ausführungsform kann das Pad als Trosttier oder Drücktier für traumatisierte Kinder in Ausnahmefällen eingesetzt werden.

Bei einer weiteren Ausführungsform kann das Pad als Kopfauflage bei z. B. eingeklemmten Personen in Seitenlage verwendet werden um diese Person am Gesicht vor weiteren Verletzungen zu schützen oder vor Hitze und Flammen.

Wie unten genauer beschrieben kann das Pad auch ein Leuchtmittel aufweisen, beispielsweise eine oder mehrere LEDs. Bevorzugt handelt es sich hierbei um wenigstens ein adaptierbares und/oder wasserdichtes und/oder stoßsicheres Leuchtmittel. So kann beispielsweise Beleuchtungsset mit mehreren, insbesondere wenigstens vier taktischen Lichtern vorgesehen sein. Diese sind bevorzugt in mehreren und insbesondere in wenigstens drei, bevorzugt wenigstens vier, bevorzugt wenigstens fünf und bevorzugt in wenigstens sechs Beleuchtungsstufen einstellbar, so dass eine Anpassung an unterschiedliche Situationen denkbar ist (beispielsweise zur Wasserrettung, bei Nacht oder zur Ausleuchtung einer Unfallstelle). Einsetzbar auch unter Wasser. Ebenso ist bevorzugt für hoheitliche Einsatzkräfte eine taktische Blendfunktion per flash light integriert.

Es kann zusätzlich ein wahlweise integrierter Sensor (etwa ein Feuchtigkeitssensor) vorgesehen sein. Dieser kann etwa eine LED bei Kontakt mit Wasser einschalten. Daneben ist es möglich, dass das Multifunktionspad eine GPS - Einheit aufweist, um dessen Lokalisierung zu erleichtern.

Weiterhin kann das Pad auch als Barriereschild zur Abwehr von Hitze und Flammen bei verschütteten oder eingeklemmten Personen verwendet werden oder als Unterlage für Retter und Einsatzkräfte bei Betreuungsgesprächen z.B. eingeklemmter Personen.

Weiterhin kommt auch eine Anwendung zum inneren Rettereinsatz beispielsweise als Bracket oder Unterlage zur Knieschonung in Frage (z. B. Scheren-, Flex- oder Spreizzangen - Einsatz).

Daneben kann das Pad auch geeignet sein, um als Warnleuchte aufgestellt zu werden (nur mit adaptierbarer LED, wahlweise mit durch Klett entfernbarer aufklappbarer LED).

Auch kann das Pad als Rucksack mit Tragevorrichtung für die Befestigung weiterer Rettungsutensilien verwendet werden und/oder mit wahlweise zusätzlichem Tragegurt ausgestattet sein.

Diese sehr umfangreichen Einsatzgebiete zeigen, dass das Pad und insbesondere seine Bestandteile wie der oben erwähnte Innenkörper sehr unterschiedlichen Anforderungen genügen müssen. Aus diesem Grund war es erforderlich, das Pad selbst, aber auch dessen Innenkörper sehr genau derart abzustimmen, dass alle oder wenigstens zahlreiche der oben erfüllten Anwendungsbeispiele ermöglicht werden. In der nachfolgenden Beschreibung wird dargelegt, wie eine derartige Abstimmung der einzelnen Bestandteile des Pads erfolgte.

Das Pad weist, wie oben erwähnt, einen Innenkörper bzw. ein Inlay auf, welches beispielsweise einen Polyurethan Kaltschaum aufweist, ggfs. mit Brandschutz und welches bevorzugt mit einer Metallschicht verklebt ist. Hierbei kann es sich beispielsweise um eine Leichtbau Aluminiumschicht handeln.

Das eigens für die obigen Anwendungsbereiche entwickelte Inlay bzw. der Innenkörper ist bei der Konfiguration zur Wasserrettung bevorzugt mit mindestens einem Foliensack umwickelt welcher wasserdicht verschlossen und insbesondere verschweißt ist. Das Pad, welches auch als IRIP-Pad oder Emergency Pad bezeichnet werden kann ist vorzugsweise eingehüllt von einem Bezug, insbesondere einem Planenbezug oder Stoffbezug. Auch dieser Bezug kann dabei Brandschutzerfordernissen entsprechen.

Bevorzugt weist dieser Bezug ein Verschließmittel, wie insbesondere einen Reißverschluss und/oder einen Klettverschluss, auf. Dieser Reißverschluss ist bevorzugt wasserdicht ausgeführt.

Vorteilhaft weist das Multifunktionspad wenigstens einen Haltegriff auf. Bei einer bevorzugten Ausführungsform sind auf einer Außenseite und insbesondere rückseitig zwei Haltegriffe vorgesehen. Diese können dabei nur als Gurt, mit einem Stoff umzogen oder auch gepolstert ausgeführt sein. Auf diese Weise wird der Einsatz zur Abwehr von Angriffen ermöglicht.

Die Haltegriffe können bevorzugt beidhändig gehalten werden oder einhändig eingesetzt werden.

Es besteht die Möglichkeit das (IRIP-Pad bzw. Emergency pad) bzw. Multifunktionspad an den Griffen zu tragen oder an außen befestigten Halteriemen oder an (insbesondere auf der Rückseite befestigten) Gurten zur Aufnahme als Rucksack zu nutzen. Ebenso besteht die Möglichkeit über vorgesehene Laschen weiteres Equipment z.B. Defibrillator oder weitere Taschen oder Rucksäcke am Pad zu befestigen.

Bevorzugt ist an wenigstens einer Seite bzw. Kante und insbesondere an der Oberkante des Multifunktionspads ein weiterer Gurt befestigt, welcher bevorzugt mittels Karabinerhaken ein Einhängen in einen Einsatzrucksack ermöglicht oder welcher, wenn das Pad als Rucksack aufgenommen wird, die Möglichkeit bietet, dort weitere Utensilien zu befestigen (wie etwa erste Hilfe Taschen, Defibrillatoren oder sonstige Ausstattung).

Bevorzugt kann das Pad in unterschiedlichen Ausstattungsstufen vom Besteller und Anwender konfiguriert und gefertigt werden, um unterschiedlichste Anwendungs- und Einsatzfälle abbilden zu können. Das IRIP-Pad Pad kann bevorzugt in allen RAL-Farben reproduziert werden und die Bedruckung als Patch kann für den jeweiligen Anwenderfall flexibel integriert werden. Das Multifunktionspad ist bevorzugt abwaschbar, zu reinigen und/oder desinfizierbar.

Bei einer weiteren bevorzugten Ausführungsform weist das Multifunktionspad eine Markierung auf, welche eine insbesondere eindeutige Identifizierung des Multifunktionspads ermöglicht. Auch wäre es möglich, dass aus dieser Markierung die Zusammensetzung des Innenkörpers ermittelt werden kann.

Bevorzugt weist das Multifunktionspad eine Länge (Erstreckung in der Längsrichtung) auf, die größer ist als 40 cm, bevorzugt größer als 50 cm, bevorzugt größer als 55 cm. Bevorzugt weist das Multifunktionspad eine Länge (Erstreckung in der Längsrichtung) auf, die kleiner ist als 100 cm, bevorzugt kleiner als 90 cm, bevorzugt kleiner als 80 cm, bevorzugt kleiner als 70 cm und besonders bevorzugt kleiner als 65 cm.

Bevorzugt weist das Multifunktionspad eine Breite (Erstreckung in der Breitenrichtung) auf, die größer ist als 20 cm, bevorzugt größer als 25 cm, bevorzugt größer als 30 cm. Bevorzugt weist das Multifunktionspad eine Breite (Erstreckung in der Breitenrichtung) auf, die kleiner ist als 70 cm, bevorzugt kleiner als 60 cm, bevorzugt kleiner als 50 cm, bevorzugt kleiner als 45 cm und besonders bevorzugt kleiner als 40 cm.

Bevorzugt weist das Multifunktionspad eine Dicke (Erstreckung in der Dickenrichtung) auf, die größer ist als 2 cm, bevorzugt größer als 4 cm, bevorzugt größer als 6 cm. Bevorzugt weist das Multifunktionspad eine Dicke (Erstreckung in der Dickenrichtung) auf, die kleiner ist als 20 cm, bevorzugt kleiner als 18 cm, bevorzugt kleiner als 16 cm, bevorzugt kleiner als 14 cm und besonders bevorzugt kleiner als 13 cm.

Bevorzugt ist eine Gesamtdicke des Innenkörpers größer als 40 mm, bevorzugt größer als 50 mm, bevorzugt größer als 60 mm, bevorzugt größer als 70 mm und besonders bevorzugt größer als 75 mm. Bevorzugt ist eine Gesamtdicke des Innenkörpers kleiner als 120 mm, bevorzugt kleiner als 110 mm, bevorzugt kleiner als 100 mm, bevorzugt kleiner als 90 mm und besonders bevorzugt kleiner als 85 mm.

Bei einer weiteren bevorzugten Ausführungsform sind der Schaumstoffkern, die Zwischenschicht und der erste Schaumstoffkörper wenigstens abschnittsweise und bevorzugt vollflächig miteinander verbunden. Bevorzugt sind der Schaumstoffkern, die Zwischenschicht und der erste Schaumstoffkörper dauerhaft und/oder nicht zerstörungsfrei trennbar miteinander verbunden.

Bevorzugt sind dabei wenigsten zwei der genannten Elemente miteinander verbunden, bevorzugt alle drei der genannten. Bevorzugt sind zwischen wenigsten zweien der Elementen Klebeschichten angeordnet, bevorzugt zwischen allen Elementen

Bevorzugt handelt es sich bei dem Klebstoff um einen Spritzklebstoff 800*223 (EG) Nr. 1272/2008 Kohlenwasserstoffe, C6-, Isoalkane, <5 % n-Hexan ist eine Mischung aus: Hexan (Isomerengemisch), Cyclopentan, n-Hexan und Pentan

Bevorzugt ist Dicke wenigstens einer der Klebeschichten und bevorzugt beider Klebeschichten größer als 0,01 mm, bevorzugt größer als 0,02 mm, bevorzugt größer als 0,03 mm und bevorzugt größer als 0,04 mm.

Bevorzugt ist die Dicke wenigstens einer der Klebeschichten und bevorzugt beider Klebeschichten geringer als 0,1 mm, bevorzugt geringer als 0,08 mm und bevorzugt geringer als 0,06 mm.

Bei einer bevorzugten Ausführungsform verläuft wenigstens eine Schicht und verlaufen beide Schichten in der Längsrichtung und in der Breitenrichtung.

Bei einer weiteren bevorzugten Ausführungsform weist der Innenkörper weiterhin einen zweiten Schaumstoffkörper auf, wobei bevorzugt der Schaumstoffkern zwischen dem ersten Schaumstoffkörper und dem zweiten Schaumstoffkörper angeordnet ist.

Bevorzugt ist eine zwischen dem Schaumstoffkern und dem zweiten Schaumstoffkörper eine zweite Zwischenschicht aus einem Kunststoff- oder Metallmaterial vorgesehen.

Besonders bevorzugt verläuft auch diese zweite Zwischenschicht parallel zu der bzw. den Außenoberflächen.

Bevorzugt ist auch diese zweite Zwischenschicht aus einem Metall gefertigt und besonderes bevorzugt eine Aluplatte. Besonders bevorzugt ist eine Dicke dieser zweiten Zwischenschicht größer als 0,1 mm, bevorzugt größer als 0,2 mm, bevorzugt größer als 0,3 mm und besonders bevorzugt größer als 0,4 mm.

Besonders bevorzugt ist eine Dicke dieser zweiten Zwischenschicht kleiner als 4,0 mm, bevorzugt kleiner als 3,5 mm, bevorzugt kleiner als 3,0 mm und besonders bevorzugt kleiner als 2,5 mm, besonders bevorzugt kleiner als 2,0 mm, besonders bevorzugt kleiner als 1,5 mm und besonders bevorzugt kleiner als 1,0 mm.

Bei einer weiteren bevorzugten Ausführungsform ist der Innenkörper symmetrisch bezüglich einer sich in der Längsrichtung und der Breitenrichtung erstreckenden Ebene aufgebaut, welche zentral durch den Schaumstoffkern verläuft.

Bei einer weiteren bevorzugten Ausführungsform ist wenigstens einer der Schaumstoffkörper aus einem Polyurethanschaum hergestellt.

Bevorzugt sind mehrere und besonders bevorzugt alle der Schaumstoffkörper bzw. Schaumstoffschichten aus einem Polyurethanschaum hergestellt.

Bei einer weiteren bevorzugten Ausführungsform weist der Schaumstoffkern eine Dicke aufweist, die größer ist als 20 mm, bevorzugt größer als 30 mm, bevorzugt größer als 40 mm und bevorzugt größer als 45 mm.

Bei einer weiteren bevorzugten Ausführungsform weist der Schaumstoffkern eine Dicke auf, die kleiner ist als 90 mm, bevorzugt kleiner als 80 mm, bevorzugt kleiner als 70 mm, bevorzugt kleiner als 60 mm und besonders bevorzugt kleiner als 50 mm.

Bevorzugt weisen die Schaumstoffelemente eine einheitliche Dicke auf.

Bei einer bevorzugten Ausführungsform weist wenigstens ein Bestandteil des Schaumstoffkörpers eine nicht entflammbare Substanz auf. Dabei ist es möglich, dass wenigstens einer der verwendeten Schaumstoffe mit einer derartigen flammhemmenden Substanz versehen und beispielsweise imprägniert ist. Bevorzugt sind mehrere der oben genannten Schaumstoffelemente mit einer flammhemmenden Substanz versehen und insbesondere imprägniert und besonders bevorzugt gilt dies für sämtliche Schaumstoffelemente.

Bei einer weiteren bevorzugten Ausführungsform weist wenigstens eines der Schaumstoffelemente ein Trockengewicht auf, welches größer ist als 30 kg/qm, bevorzugt größer als 40 kg/qm bevorzugt größer als 45 gk/qm und besonders bevorzugt größer als 50 kg/qm.

Bei einer weiteren bevorzugten Ausführungsform weist wenigstens eines der Schaumstoffelemente ein Trockengewicht auf, welches kleiner ist als 80 kg/qm, bevorzugt kleiner als 70 kg/qm bevorzugt kleiner als 65 gk/qm und besonders bevorzugt kleiner als 60 kg/qm.

Bei einer weiteren vorteilhaften Ausführungsform ist der Bezug aus einem Textilmaterial hergestellt.

Bei einer weiteren bevorzugten Ausführungsform ist der Bezug wenigstens abschnittsweise mit dem Innenkörper vernäht.

Bei einer weiteren vorteilhaften Ausführungsform weist der Bezug eine Dicke auf, die größer ist als 0,2 mm, bevorzugt größer als 0,4 mm und bevorzugt größer als 0,6 mm

Bei einer weiteren bevorzugten Ausführungsform weist der Bezug eine Dicke auf, die kleiner ist als 2,0 mm, bevorzugt kleiner als 1,8 mm, bevorzugt kleiner als 1,6 mm, bevorzugt kleiner als 1,4 mm, bevorzugt kleiner als 1,2 mm und besonders bevorzugt kleiner als 1,0 mm und besonders bevorzugt kleiner als 0,8 mm.

Besonders bevorzugt weist wenigstens einer der verwendeten Schaumstoffe (und insbesondere der Schaumstoff aus dem der Schaumstoffkern hergestellt ist und/oder der Schaumstoff, aus dem der oder die Schaumstoffkörper hergestellt sind) eine Stauchhärte auf, die größer ist als 4,0 bevorzugt größer als 4,5, bevorzugt größer als 5,0.

Besonders bevorzugt weist wenigstens einer der verwendeten Schaumstoffe (und insbesondere der Schaumstoff aus dem der Schaumstoffkern hergestellt ist und/oder der Schaumstoff, aus dem der oder die Schaumstoffkörper hergestellt sind) eine Stauchhärte auf, die kleiner ist als 9,0 bevorzugt kleiner als 8,0 bevorzugt kleiner als 7,0.

Besonders bevorzugt weist der Schaumstoff eine Zugfestigkeit zwischen 60 kpa und 80 kpa und bevorzugt zwischen 65 kpa und 75 kpa auf.

Besonders bevorzugt wird als Zwischenschicht Leichtbau Aluminium DX51D und Z285 MB verwendet.

Das eigens dafür entwickelte Inlay ist bevorzugt bei der Konfiguration zur Wasserrettung mit einem wahlweise 2 Foliensäcken umwickelt welcher wasserdicht verschweißt ist. Das IRIP-Pad bzw. Emergency Pad ist bevorzugt eingehüllt von einem Stoffbezug.

Dieser Stoffbezug weist bevorzugt eine Grammatur auf, die größer ist als 500 g/m², bevorzugt größer als 550 g/m² , bevorzugt größer als 600 g/m² , bevorzugt größer als 650 g/m², bevorzugt größer als 700 g/m² , bevorzugt größer als 750 g/m² und besonders bevorzugt größer als 800 g/m² .

Weiterhin weist der Stoffbezug bevorzugt eine Grammatur auf, die kleiner ist als 1000 g/m², bevorzugt kleiner als 950 g/m² , bevorzugt kleiner als 900 g/m² , bevorzugt kleiner als 850 g/m².

Bei einer weiteren bevorzugten Ausführungsform ist zwischen dem Innenkörper und dem Bezug wenigstens eine weitere Schicht und insbesondere eine flexible Schicht angeordnet. Bevorzugt ist diese weitere Schicht wenigstens aus einer Schaumstoffschicht und einer Vliesschicht zusammengesetzt.

Bevorzugt handelt es sich hierbei um eine Schaum-Vlieskombination, die bevorzugt durch Vernadeln der Faserschicht mit einem Schaum hergestellt ist.

Bevorzugt weist diese weitere Schicht eine Dicke auf, die größer ist als 0,5 mm, bevorzugt größer als 0,8 mm, bevorzugt größer als 1,0 mm, bevorzugt größer als 1,2 mm und besonders bevorzugt größer als 1,4 mm.

Bevorzugt weist diese weitere Schicht eine Dicke auf, die kleiner ist als 3,0 mm, bevorzugt kleiner als 2,5 mm, bevorzugt kleiner als 2,0 mm, bevorzugt kleiner als 1,8 mm und besonders bevorzugt kleiner als 1,6 mm.

Bei einer weiteren bevorzugten Ausführungsform ist an wenigstens einer Außenoberfläche des Multifunktionspads wenigstens eine Tasche angeordnet und insbesondere angenäht. Insbesondere ist an einer Rückseite und/oder an wenigstens einer Außenoberfläche wenigstens ein Griffelement angeordnet.

Bei einer weiteren bevorzugten Ausführungsform ist wenigstens eine weitere bzw. zusätzliche Tasche insbesondere lösbar an dem Multifunktionspad vorgesehen. Bevorzugt ist diese weitere Tasche mittels wenigstens einem lösbaren Verbindungselement an dem Multifunktionspad und insbesondere an einer Unterseite desselben vorgesehen.

Bevorzugt handelt es sich bei diesem lösbaren Verbindungselement um eine Steckverbindung. Auf diese Weise kann die zusätzliche Tasche an dem Pad und insbesondere an dessen Unterseite lösbar befestigt werden

Bei einer weiteren bevorzugten Ausführungsform weist das Multifunktionspad mehrere an diesem angeordnete und insbesondere angenähte Taschen auf.

Bei einer weiteren bevorzugten Ausführungsform weist das Multifunktionspad wenigstens einen und bevorzugt mehrere Griffe auf. Auch diese Griffe können dabei bevorzugt an den Bezug angenäht sein.

Bei einer weiteren bevorzugten Ausführungsform weist das Multifunktionspad wenigstens ein Gurtelement und insbesondere wenigstens einen Haltegurt auf.

Bevorzugt sind alle der genannten Teile miteinander stabil vernäht. Die Taschen sind bevorzugt an der Rückseite des Multifunktionspads vernäht und können besonders bevorzugt beliebig angepasst und erweitert werden.

Besonders bevorzugt ist das Multifunktionspad an der EH Tasche unten bedruckt und/oder beschriftet. Diese Bedruckung der Schriften und Reflektoren erfolgt bevorzugt durch Foliendruck und/oder wird aufgebügelt.

Bevorzugt sind mehrere und bevorzugt alle Haltegurte in den Nähten befestigt und/oder verstärkt.

Bei einer bevorzugten Ausführungsform ist ein oberer Haltegurt vorgesehen. Dieser obere Haltegriff ist bevorzugt mittels eines Quergurts vernäht und bevorzugt zweimal vernäht, um die Wasserrettung und Eisrettung zu ermöglichen. Bevorzugt ist ein weiterer Gurt auf der Innenseite eingebracht.

Bei einer weiteren vorteilhaften Ausführungsform ist der Innenkörper in einer Hülle angeordnet. Besonders bevorzugt ist der innerhalb der Hülle angeordnete Innenkörper wiederum innerhalb des Bezugs angeordnet.

Bei einer weiteren bevorzugten Ausführungsform ist diese Hülle wasserdicht ausgebildet und insbesondere wasserdicht verschweißt. Bevorzugt ist diese Hülle aus einem Kunststoff hergestellt.

Bei einer weiteren bevorzugten Ausführungsform ist das Multifunktionspad schwimmfähig und/oder wenigstens teilweise wasserfest.

Das Multifunktionspad kann mit oder ohne Schwimmfunktion ausgestattet sein. Ohne Schwimmfunktion entfällt der innere Foliensack bzw. die oben erwähnte Hülle. Es ist weiterhin möglich, dass Multifunktionspad mit oder ohne ein Schwimmseil vorzusehen.

Sofern das Multifunktionspad ohne Schwimmseil eingesetzt wird kann jegliches zugelassene Rettungsseil an dem Multifunktionspad befestigt werden und bevorzugt an einem Haltegriff und insbesondere am oberen Haltegriff befestigt werden, was insbesondere mittels eines Karabiners erfolgen kann.

Bevorzugt weist ausschließlich der obere Haltegriff die Stabilität zur Personenbergung und Rettung auf. Bevorzugt ergibt sich die Haptik und die Festigkeit aus dem Nähprozess und/oder ist durch den Bezug gegeben.

Bei einer weiteren vorteilhaften Ausführungsform ist das Multifunktionspad bezüglich wenigstens einer Symmetrieebene symmetrisch aufgebaut. Bevorzugt handelt es sich hierbei um eine Symmetrieebene, die sich in der Längsrichtung und in der Breitenrichtung erstreckt. Bei einer weiteren bevorzugten Ausführungsform weist das Multifunktionspad abgerundete Ecken und/oder Kanten auf. Bei einer weiteren bevorzugten Ausführungsform weist das Multifunktionspad abgeschrägte Kanten auf.

Bei einer weiteren bevorzugten Ausführungsform weist das Multifunktionspad einen ersten Abschnitt mit einer ersten Dicke auf und einen zweiten Abschnitt mit einer zweiten Dicke, welche von der ersten Dicke abweicht.

Bevorzugt geht der erste Abschnitt in den zweiten Abschnitt in einem Übergangsabschnitt mit einer sich ändernden Dicke und insbesondere einer sich linear ändernden Dicke über.

Bei einer weiteren vorteilhaften Ausführungsform ist das Multifunktionspad kugelsicher ausgebildet. Insbesondere ist bevorzugt das Multifunktionspad dazu geeignet und bestimmt, Geschosse von Pistolen oder auch von Maschinepistolen abzufangen und so den Benutzer zu schützen.

Besonders bevorzugt ist das Multifunktionspad im Wesentlichen über seine gesamte Oberfläche oder nahezu seine gesamte Oberfläche hinweg kugelsicher ausgebildet. Unter der nahezu gesamten Oberfläche wird dabei ein Oberflächenanteil verstanden der wenigstens 80% der gesamten Oberfläche, bevorzugt wenigstens 85% der gesamten Oberfläche, bevorzugt wenigstens 90% der gesamtem Oberfläche und bevorzugt wenigstens 95% der gesamten Oberfläche beträgt.

Bevorzugt weist das Multifunktionspad wenigstens eine Ballistikschicht auf, welche diese Kugelsicherheit bewirkt. Besonders bevorzugt handelt es sich bei dieser Ballistikschicht um eine Schicht, welche keine Außenoberfläche des Multifunktionspads ausbildet und/oder welche im Inneren des Multifunktionspads angeordnet ist.

Bevorzugt ist an wenigstens einer Oberfläche dieser Ballistikschicht eine Klebeschicht und besonders bevorzugt sind an zwei Oberflächen und insbesondere zwei einander gegenüberliegenden Oberflächen dieser Ballistikschicht Klebeschichten angeordnet.

Besonders bevorzugt ist diese Ballistikschicht zwischen zwei Schaumstoffschichten angeordnet.

Bevorzugt weist die Ballistikschicht wenigstens eine Ballistikmatte und bevorzugt wenigstens eine Weichballistikmatte auf.

Bei einer weiteren bevorzugten Ausführungsform weist die Ballistikschicht wenigstens zwei Ballistikmatten und bevorzugt wenigstens zwei Weichballistikmatten auf. Bevorzugt sind diese zwei Weichballistikmatten aneinander angeordnet.

Bevorzugt sind diese beiden Ballistikmatten in unterschiedlichen Richtungen ausgerichtet Dies wird durch die Verwteppung im Winkel von 45Grad und 90 Grad erreicht, welche übereinander gelegt werden

Bei einer weiteren vorteilhaften Ausführungsform ist eine Matte in einem Winkel gegenüber der vertikalen Richtung des Pads angeordnet, der zwischen 70° und 110°, bevorzugt zwischen 80° und 100° und besonders bevorzugt zwischen 85° und 95° liegt.

Bei einer weiteren vorteilhaften Ausführungsform ist eine Matte in einem Winkel gegenüber der vertikalen Richtung des Pads angeordnet, der zwischen 25° und 65°, bevorzugt zwischen 30° und 60° und besonders bevorzugt zwischen 35° und 55° liegt.

Bevorzugt ist die wenigstens eine Ballistikmatte in einer Einschweißung angeordnet und/oder in einer Hülle verschweißt. Besonders bevorzugt handelt es sich dabei um eine wasserdichte Einschweißung.

Besonders bevorzugt sind beide oder allgemein alle Ballistikmatten in einer insbesondere gemeinsamen Einschweißung angeordnet und/oder in einer Hülle verschweißt. Besonders bevorzugt handelt es sich dabei um eine wasserdichte Einschweißung und/oder Hülle.

Bei einer weiteren bevorzugten Ausführungsform sind die Ballistikmatte(n) und die Umhüllung bzw. Einschweißung, in der sich die Ballistikmatte(n) befindet oder befinden als Einheit ausgebildet. Dadurch ist es möglich, dass das Multifunktionspad auf Wunsch durch Hinzufügung dieser Einheit in einer kugelsicheren Version zur Verfügung gestellt werden kann.

Bevorzugt sind die Ballistikmatten miteinander verbunden und insbesondere miteinander in einem Bezug eingelegt. Vorzugsweise sind die Elemente wasserdicht verschweißt

Besonders bevorzugt handelt es sich bei der Einschweißung um ein Gewebe und insbesondere um ein Gewebe aus Para-Aramid.

Besonders bevorzugt weist die Ballistikschicht ein Gewicht auf, welches größer ist als 100g, bevorzugt größer als 200g und besonders bevorzugt größer als 300g.

Bei einer weiteren bevorzugten Ausführungsform, weist die Ballistikschicht ein Gewicht auf, welches geringer ist als 1000g, bevorzugt geringer als 800g, bevorzugt geringer als 600g und besonders bevorzugt geringer als 500g.

Das Gewicht sollte einerseits möglichst gering sein, um die Tragbarkeit des Pads nicht übermäßig zu beeinträchtigen. Andererseits sollte die Ballistikschicht jedoch einen sicheren Schutz gegenüber Angriffen mit Schusswaffen nach VPAM Klasse 3+ bieten.

Bei einer weiteren bevorzugten Ausführungsform entfällt bei der Verwendung der Ballistikschicht wenigstens eine Hako-Flexeinlage und entfallen bevorzugt beide Hako-Flexeinlagen.

Bei einer weiteren bevorzugten Ausführungsform weist die Ballistikschicht eine Dicke auf, die größer ist als 8mm bevorzugt größer als 9mm und besonders bevorzugt größer als 10 mm ist.

Bei einer weiteren bevorzugten Ausführungsform weist die Ballistikschicht eine Dicke auf, die kleiner ist als 13mm bevorzugt kleiner als 12mm und besonders bevorzugt kleiner als 11mm.

Bei einer weiteren bevorzugten Ausführungsform ist die Ballistikschicht in den Schaumstoffkern eingebettet. So kann die Ballistikschicht im Wesentlichen innerhalb des Schaumstoffkerns angeordnet sein.

Bei einer weiteren vorteilhaften Ausführungsform das Multifunktionspad wenigstens zwei, bevorzugt wenigstens drei Patches auf.

Bevorzugt ist wenigstens eines dieser Patches und besonders bevorzugt sind mehrere und bevorzugt alle dieser Patches mittels lösbarer Verbindungsmittel und insbesondere mittels Klettverbindungen befestigbar.

Bei einer weiteren bevorzugten Ausführungsform sind diese Patches mit Reflektionselementen und/oder mit Einheitsbezeichnungen der Anwendungskräfte und/oder mit Logos kennzeichenbar.

Diese Elemente können dabei bevorzugt an den Patches aufgeklebt, angenäht oder in sonstiger Weise befestigt sein.

Weitere Vorteile und Ausführungsformen ergeben sich aus den beigefügten Zeichnungen:
Darin zeigen:
- Fig. 1: eine rückseitige Ansicht eines erfindungsgemäßen Multifunktionspads;
- Fig. 2: eine Vorderansicht eines erfindungsgemäßen Multifunktionspads;
- Fig. 3: eine Seitenansicht eines erfindungsgemäßen Multifunktionspads;
- Fig. 4: eine Seitenansicht eines erfindungsgemäßen Multifunktionspads;
- Fig. 5: einen Schichtaufbau eines erfindungsgemäßen Multifunktionspads;
- Fig. 6: einen weiteren Schichtaufbau eines erfindungsgemäßen Multifunktionspads; und
- Fig. 7: eine Darstellung einer Ballistikschicht für das Multifunktionspad aus Fig. 6.

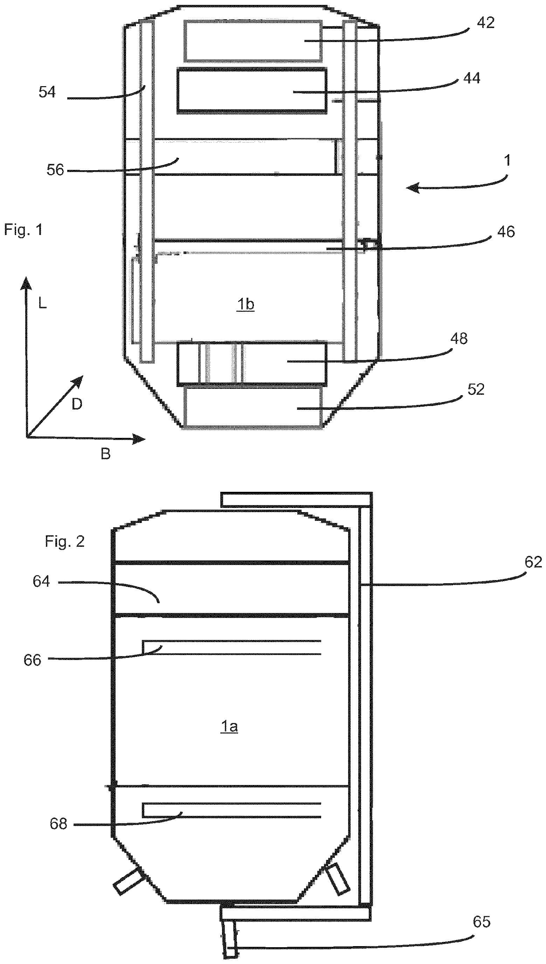

Fig. 1 zeigt eine Rückseite 1b eines erfindungsgemäßen Multifunktionspads. Diese Rückseite weist eine Vielzahl von an dieser angeordnete Funktionseinheiten auf. Weiterhin ist bevorzugt eine Anleitung, insbesondere eine EH Anleitung vorgesehen. Diese befindet sich bevorzugt auf der Höhe des Bezugszeichens. Es handelt sich hierbei bevorzugt um eine wechselbare (EH) Anleitung, die besonders bevorzugt unter einem Sichtfenster aus Kunststoffangeordnet ist, welches bevorzugt geöffnet und besonders bevorzugt mittels Klettverschluss geöffnet und bevorzugt upgedatet werden kann. Diese Funktionseinheiten sind dabei optional und können in Abhängigkeit von der Konfiguration des Multifunktionspads unterschiedlich sein.

Das Bezugszeichen L kennzeichnet eine Längsrichtung, in der sich das Multifunktionsband erstreckt, das Bezugszeichen B eine Breitenrichtung und das Bezugszeichen D eine Dickenrichtung.

Das Bezugszeichen 42 kennzeichnet eine erste Tasche. Diese kann beispielsweise zur Aufnahme eines Schwimmseils dienen, welches zur Rettung aus Gewässern dienen kann.

Das Bezugszeichen 44 kennzeichnet ein erstes Griffelement bzw. Halteelement. Das Bezugszeichen 56 kennzeichnet ein - Befestigungsband.

Das Bezugszeichen 54 kennzeichnet einen optionalen Gurt, der beispielsweise als Rucksackgut dienen kann. Dieser verläuft bevorzugt in der Längsrichtung L.

Das Bezugszeichen 52 kennzeichnet eine zweite Tasche, welche beispielsweise als EH Aufnahmetasche für Artikel wie Decken, Masken oder Handschuhe oder auch für sonstige Artikel dienen kann. Das Bezugszeichen 48 kennzeichnet einen weiteren Griff oder Halter.

Das Bezugszeichen 46 kennzeichnet einen optionalen Reflektor.

Fig. 2 zeigt eine Vorderansicht des in Fig. 1 gezeigten Multifunktionspads. Dabei bezieht sich das Bezugszeichen 62 auf einen optionalen Umhängegurt. Die Bezugszeichen 66 und 68 kennzeichnen zwei weitere Reflektionsstreifen, welche auf der Vorderseite 1a angeordnet sind.

Das Bezugszeichen 65 kennzeichnet einen Befestigungsgurt für weitere Ausstattung. Dieser Befestigungsgurt kann dabei an einer Seitenwand des Multifunktionspads angeordnet sein.

Optional ist es möglich, Beleuchtungseinrichtungen an einer Vorderwand 1a zu befestigen. Diese Befestigung ist beispielsweise mittels Klettverschlüssen an dem Bestandstoff oder auch an einem der genannten Gurte möglich. Die Beleuchtung kann als stand alone Lösung beispielsweise nur mit Klett in Metallgehäuse als auch als Gehäuse innerhalb eines Bezuges am Pad befestigt werden oder an anderen Multifunktionskleidungen oder Vorrichtungen.

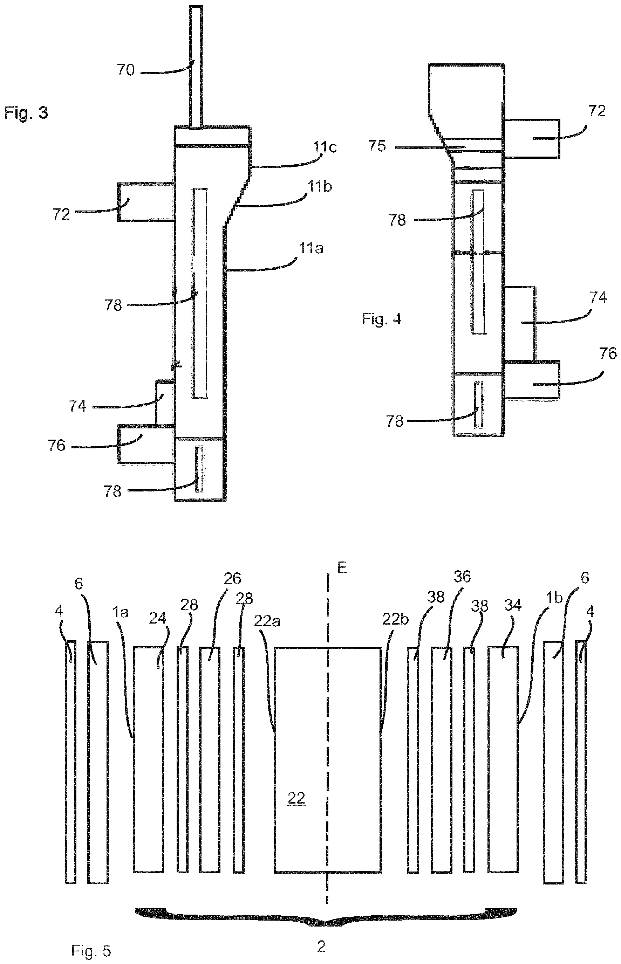

Die Fig. 3 und 4 zeigen zwei Seitenansichten des Multifunktionspads. Dabei beziehen sich die Bezugszeichen 78 wieder auf optionale Reflektionsstreifen, (wahlweise) welche an diesen Seitenflächen angeordnet und insbesondere angenäht sind.

Fig. 5 zeigt einen Schichtaufbau eines erfindungsgemäßen Multifunktionspads. Dabei bezieht sich das Bezugszeichen 2 auf einen Innenkörper, der innerhalb eines Bezugs 4 angeordnet ist. Dieser Innenkörper ist wiederum mehrschichtig aufgebaut.

Im Zentrum weist der Innenkörper 2 einen Schaumstoffkern 22 auf, der hier eine Dicke zwischen 43 und 48 mm aufweist. Dabei kann dieser Schaumstoff imprägniert sein, etwa mit einem Flammschutzmittel. Es kann sich jedoch auch um einen an sich brandschutzgeprüften Schaumstoff handeln.

Das Bezugszeichen 24 kennzeichnet einen ersten Schaumstoffkörper und das Bezugszeichen 34 einen zweiten Schaumstoffkörper, welche jeweils parallel zu dem Schaumstoffkern angeordnet sind. Diese beiden Schaumstoffkörper weisen jeweils eine Dicke auf, die zwischen 15 mm und 20 mm liegt.

Zwischen dem ersten Schaumstoffkörper 24 und dem Schaumstoffkern 22 ist eine erste Zwischenschicht 26 angeordnet. Bei dieser Schicht handelt es sich bevorzugt um eine metallische Schicht und insbesondere um eine Aluplatte bzw. ein Alublech. Diese Aluplatte weist bevorzugt eine Dicke auf, die zwischen 0,8 mm und 1,2 mm. liegt. Diese Zwischenschicht kann einen Schutz gegen Angriffe mit Stichwaffen bieten.

Zwischen dem zweiten Schaumstoffkörper 34 und dem Schaumstoffkern 22 ist eine zweite Zwischenschicht 35 angeordnet. Bei dieser Schicht handelt es sich bevorzugt um eine metallische Schicht und insbesondere um eine Aluplatte bzw. ein Alublech. Diese Aluplatte weist bevorzugt eine Dicke auf, die zwischen 0,8 mm und 1,2 mm. liegt. Diese Zwischenschicht kann ebenfalls einen Schutz gegen Angriffe mit Stichwaffen bieten.

Diese einzelnen Schichten d.h. die Schaumstoffkörper, die Zwischenschichten und der Schaumstoffkern sind bevorzugt mittels Klebstoffschichten 28 verbunden. Diese Klebstoffschichten können beispielsweise aus einem Acrylatklebstoff bestehen. Die Klebeschichten weisen bevorzugt Schichtdicken auf, die zwischen 0,03 mm und 0,07 mm liegen.

Die Bezugszeichen 4 kennzeichnen den Bezug, der bevorzugt den Innenkörper 2 vollständig umgibt. Das Bezugszeichen 6 kennzeichnet eine weitere optionale Schicht, insbesondere eine sog. Hakoflex-Schicht.

Das Bezugszeichen E kennzeichnet eine Mittelebene, welche hier gleichzeitig eine Symmetrieebene bezüglich des Innenkörpers darstellt. Dies bedeutet, dass bevorzugt der Innenkörper symmetrisch mit dem Schaumstoffkern im Zentrum aufgebaut ist.

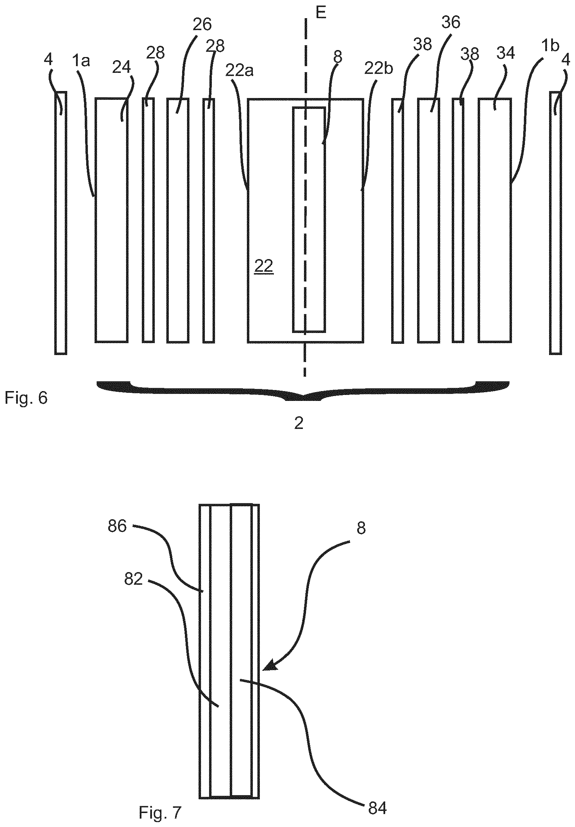

Fig. 6 zeigt eine weitere Ausgestaltung eines erfindungsgemäßen Pads bzw. dessen Schichtaufbau. Bei diesem Aufbau ist eine weitere insbesondere zentrale Ballistikschicht vorgesehen, welche zum Abfangen von Geschossen von Schusswaffen dient. Diese zentrale Ballistikschicht ist bevorzugt innerhalb des Schaumstoffkerns 22 angeordnet.

Bevorzugt weist diese Ballistikschicht, wie in Fig. 7 gezeigt, wenigstens zwei und bevorzugt genau zwei Ballistikmatten 82, 84 auf, die bevorzugt aneinander angeordnet sind. Diese Ballistikmatten sind dabei in unterschiedlichen Winkeln ausgerichtet. Besonders bevorzugt sind diese beiden Ballistikmatten flächig aneinander angeordnet. Besonders bevorzugt weist die Ballistikschicht wenigstens eine Klebeschicht auf, mit der sie an benachbarten Schichten angeklebt ist.

Das Bezugszeichen 86 kennzeichnet eine Umhüllung bzw. Einschweißung, innerhalb derer die beiden Ballistikmatten 82, 84 angeordnet sind.

Anders als bei der oben in Fig. 5 gezeigten Ausführungsform ist bei der in Fig. 6 gezeigten Ausführungsform keine Hakoflex - Schicht 6 vorgesehen. Auf diese Weise kann Gewicht und Dicke eingespart werden.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Multifunktionspad (1), insbesondere für Feuerwehren, Polizeieinheiten, Rettungsdienste und dergleichen mit einem Innenkörper (2) der sich in einer Längsrichtung (L), einer zu der Längsrichtung senkrecht stehenden Breitenrichtung (B) und einer zu der Längsrichtung und der Breitenrichtung senkrecht stehenden Dickenrichtung erstreckt, wobei die Erstreckung in der Längsrichtung und die Erstreckung in der Breitenrichtung wesentlich größer ist als die Erstreckung in der Dickenrichtung, wobei das Multifunktionspad eine erste Außenoberfläche (1a) und eine zweite Außenoberfläche (1b) aufweist und die zweite Außenoberfläche (1b) der erste Außenoberfläche (1a) gegenüber liegt und wobei dieser Innenkörper wenigstens teilweise von einem flexiblen Bezug (4) umhüllt ist,
**dadurch gekennzeichnet, dass**
der Innenkörper (2) mehrschichtig aufgebaut ist und einen Schaumstoffkern (22) aufweist, der sich ebenfalls in der Längsrichtung (L), der Breitenrichtung (B) und der Dickenrichtung (D) erstreckt und der eine erste Außenoberfläche (22a) und eine zweite Außenoberfläche (22b) aufweist, wobei die erste Außenoberfläche (22a) der zweiten Außenoberfläche (22b) gegenüberliegt und der Innenkörper weiterhin einen ersten Schaumstoffkörper (24) sowie eine zwischen diesem Schaumstoffkern (22) und dem ersten Schaumstoffkörper (24) angeordnete erste Zwischenschicht (26) aus einem Kunststoff- oder Metallmaterial aufweist.

2. Multifunktionspad nach Anspruch 1,
**dadurch gekennzeichnet, dass**
der Schaumstoffkern (22), die Zwischenschicht (26) und der erste Schaumstoffkörper (24) wenigstens abschnittsweise miteinander verbunden sind.

3. Multifunktionspad nach wenigstens einem der vorangegangenen Ansprüche,
**dadurch gekennzeichnet, dass**
der Innenkörper (2) weiterhin einen zweiten Schaumstoffkörper (34) aufweist, wobei der Schaumstoffkern zwischen dem ersten Schaumstoffkörper (24) und dem zweiten Schaumstoffkörper (34) angeordnet ist und bevorzugt eine zwischen dem Schaumstoffkern (22) und dem zweiten Schaumstoffkörper (34) angeordnete zweite Zwischenschicht (26) aus einem Kunststoff- oder Metallmaterial vorgesehen ist.

4. Multifunktionspad nach wenigstens einem der vorangegangenen Ansprüche,
**dadurch gekennzeichnet, dass**
wenigstens einer der Schaumstoffkörper aus einem Polyurethanschaum hergestellt ist und/oder wenigstens ein Schaumstoffkörper eine Dicke aufweist, die größer ist als 5 mm, bevorzugt größer als 10 mm, bevorzugt größer als 15 mm und/oder wenigstens ein Schaumstoffkörper eine Dicke aufweist, die kleiner ist als 40 mm, bevorzugt kleiner als 30 mm, bevorzugt kleiner als 25 mm und besonders bevorzugt kleiner als 20 mm.

5. Multifunktionspad nach wenigstens einem der vorangegangenen Ansprüche,
**dadurch gekennzeichnet, dass**
der Schaumstoffkern (22) eine Dicke aufweist, die größer ist als 20 mm, bevorzugt größer als 30 mm, bevorzugt größer als 40 mm und bevorzugt größer als 45 mm und/oder der Schaumstoffkern (22) eine Dicke aufweist, die kleiner ist als 90 mm, bevorzugt kleiner als 80 mm, bevorzugt kleiner als 70 mm, bevorzugt kleiner als 60 mm und besonders bevorzugt kleiner als 50 mm.

6. Multifunktionspad nach wenigstens einem der vorangegangenen Ansprüche,
**dadurch gekennzeichnet, dass**
wenigstens ein Bestandteil des Schaumstoffkörpers eine nicht entflammbare Substanz aufweist.

7. Multifunktionspad nach wenigstens einem der vorangegangenen Ansprüche,
**dadurch gekennzeichnet, dass**
der Bezug aus einem Textilmaterial hergestellt und/oder der Bezug eine Dicke aufweist, die größer ist als 0,2 mm, bevorzugt größer als 0,4 mm und bevorzugt größer als 0,6 mm und/oder der Bezug eine Dicke aufweist, die kleiner ist als 2,0 mm, bevorzugt kleiner als 1,8 mm, bevorzugt kleiner als 1,6 mm, bevorzugt kleiner als 1,4 mm, bevorzugt kleiner als 1,2 mm und besonders bevorzugt kleiner als 1,0 mm und besonders bevorzugt kleiner als 0,8 mm.

8. Multifunktionspad nach wenigstens einem der vorangegangenen Ansprüche,
**dadurch gekennzeichnet, dass**
an wenigstens einer Außenoberfläche wenigstens eine Tasche angeordnet und insbesondere angenäht ist, insbesondere der Rückseite und/oder an wenigstens einer Außenoberfläche wenigstens ein Griffelement angeordnet ist und/oder wenigstens eine mittels Steckverbindern adaptierbare zusätzliche Tasche -insbesondere an der Unterseite - vorgesehen ist.

9. Multifunktionspad nach wenigstens einem der vorangegangenen Ansprüche,
**dadurch gekennzeichnet, dass**
der Innenkörper in einer Hülle angeordnet ist und bevorzugt diese Hülle wasserdicht ausgebildet und insbesondere wasserdicht verschweißt ist.

10. Multifunktionspad nach wenigstens einem der vorangegangenen Ansprüche,
**dadurch gekennzeichnet, dass**
das Multifunktionspad schwimmfähig und/oder wenigstens teilweise wasserfest ist.

11. Multifunktionspad nach wenigstens einem der vorangegangenen Ansprüche,
**dadurch gekennzeichnet, dass**
das Multifunktionspad wenigstens ein Leuchtmittel aufweist, welches besonders bevorzugt an einer Außenoberfläche des Multifunktionspads angeordnet ist.

12. Multifunktionspad nach wenigstens einem der vorangegangenen Ansprüche,
**dadurch gekennzeichnet, dass**
das Multifunktionspad bezüglich wenigstens einer Symmetrieebene symmetrisch aufgebaut ist.

13. Multifunktionspad nach wenigstens eine der vorangegangenen Ansprüche,
**dadurch gekennzeichnet, dass**
das Multifunktionspad einen ersten Abschnitt mit einer ersten Dicke aufweist und einen zweiten Abschnitt mit einer zweiten Dicke, welche von der ersten Dicke abweicht und bevorzugt der erste Abschnitt in den zweiten Abschnitt in einem Übergangsabschnitt mit einer sich ändernden Dicke übergeht.

14. Multifunktionspad nach wenigstens einem der vorangegangenen Ansprüche,
**dadurch gekennzeichnet, dass**
das Multifunktionspad wenigstens abschnittsweise kugelsicher ausgebildet ist.

15. Multifunktionspad nach wenigstens einem der vorangegangenen Ansprüche,
**dadurch gekennzeichnet, dass**
das Multifunktionspad wenigstens zwei, bevorzugt wenigstens drei Patches aufweist, welche bevorzugt mittels Klettverbindungen befestigbar sind, wobei bevorzugt diese Patches mit Reflektionselementen und/oder mit Einheitsbezeichnungen der Anwendungskräfte und/oder mit Logos kennzeichenbar sind.
